# FASCICULE DE BREVET EUROPEEN

(11) **EP 0 681 488 B1**
(45) Date de publication et mention de la délivrance du brevet: **22.01.1997**
(21) Numéro de dépôt: 93901773.7
(22) Date de dépôt: 25.11.1992
(51) Int. Cl.: A61M 5/148

(54) **APPAREIL ELECTRONIQUE POUR LA TRANSFUSION DU SANG**
ELEKTRONISCHE BLUTTRANSFUSIONSVORRICHTUNG
ELECTRONIC APPARATUS FOR BLOOD TRANSFUSION

(30) Priorité: 03.12.1991 FR 9114940
(43) Date de publication de la demande: 15.11.1995
(73) Titulaire: LE BOEUF, Guy, F-86800 Lavoux (FR)
(72) Inventeur: LE BOEUF, Guy, F-86800 Lavoux (FR)
(74) Mandataire: Dawidowicz, Armand
(86) Numéro de dépôt international: FR9201098
(87) Numéro de publication internationale: WO9310832

(56) Documents cités:
- EP-A- 0 019 554
- WO-A-88/07384
- US-A- 3 902 635
- US-A- 4 626 243
- US-A- 4 747 826

## Description

Depuis le début des transfusions de sang, leur technique s'est améliorée. Il y eu d'abord la conservation du sang dans les poches stériles d'environ 300 cm³ à une température de + 4°C.

Depuis quelques années, les centres de transfusion séparent les globules rouges du plasma, ce qui permet de ne transfuser que la partie utile du sang, soit les concentrés globulaires (hématies) soit le plasma ou du sérum. Des appareils d'aide à la transfusion sont aussi apparus, à savoir :
1/ des pompes accélératrices de sang, pour remédier le plus rapidement possible à une hémorragie qui, lorsqu'elle est massive peut conduire à la mort. Ces pompes du type péristaltique d'abord manuelles ont été par la suite commandées par un moteur électrique à vitesse variable réglée électroniquement.
2/ des réchauffeurs de sang : les médecins se sont aperçu que les transfusions massives de sang froid provoquaient environ 8% de mortalité qui est évitée avec du sang réchauffé à 37°C. De plus, des études cliniques ont montré que la transfusion avec du sang réchauffé diminuait le volume transfusé d'environ 30%, ce qui diminue les risques d'infection (SIDA, Hépatite B, etc.) et est plus confortable pour le patient qui ne frissonne plus après l'intervention.
3/ d'autre part, le sang conservé possède des microagrégats et des impuretés qu'il est souhaitable d'éliminer (car ils obstruent les vaisseaux sanguins de surface). En plus du filtre classique de 173 microns, des filtres dits à micro-filtration de 40 microns sont utilisés.

Actuellement une ligne de transfusion comporte donc :
1/ un sac de concentré globulaire, (alternativement un sac de plasma ou de soluté),
2/ un filtre de 175 microns,
3/ un micro-filtre de 40 microns,
4/ une pompe accélératrice de sang,
5/ un réchauffeur de sang,
6/ une tubulure à usage unique pour la pompe,
7/ une poche à usage unique pour le réchauffeur de sang (dite poche à sang) et,
8/ une ligne de perfusion reliant le tout raccordée par des embouts coniques de type Luer et un trocart introduit dans la veine du patient.

La mise en service de ces différents éléments est longue et le raccordement de ceux-ci peut parfois provoquer des prises d'air qu'il faut absolument éliminer car une introduction dans les veines de quelques cm³ d'air est mortelle.

D'autre part, tous ces matériels prennent beaucoup de place à proximité de la table d'opération qui doit être dégagée le plus possible.

Toutes les pompes actuellement utilisées sont du type péristaltique soit classique avec un flux rétrograde lors du brusque gonflement de la tubulure de sortie au moment du dégagement du galet, soit sans flux rétrograde.

Les pompes qui ont un flux rétrograde nécessitent un détecteur électronique de présence d'air qui arrête la pompe en cas d'intrusion d'air. Avec les pompes à flux continu, ayant un débit qui va toujours dans le même sens, les bulles d'air peuvent être piégées dans un appareil dit piège à bulles d'air, ce qui n'est pas possible dans le cas d'un flux rétrograde (ou alternatif) car les pièges à air se remplissent alors d'air qui se mélange au sang sous l'effet du flux rétrograde lors de chaque remontée du liquide transfusé.

Par ailleurs, la pompe et le réchauffeur possèdent chacun des instruments de réglage et de contrôle ainsi que des sécurités et alarmes (lumineuses et sonores).

La présente invention a pour but la réalisation d'un appareil de transfusion compact comprenant :
1/ une première pompe de conception nouvelle pour transfuser le concentré globulaire (ou le sang complet).
2/ une seconde pompe identique pour la transfusion de plasma, de soluté, d'albumine ou de sang complet.
3/ un réchauffeur de sang réchauffant les liquides mélangés provenant des deux pompes.
4/ un filtre à micro-filtration avec un piège à air.
5/ une ligne de transfusion à usage unique comprenant : deux tubulures d'entrée (une pour le concentré et une pour le plasma, le soluté, l'albumine ou le sang complet) avec cônes Luer, la poche spécifique pour le réchauffeur de sang, le micro-filtre, le piège à air, un cône Luer et le trocart en fin de la tubulure de perfusion.

Tous ces composants sont regroupés dans un seul appareil compact afin d'améliorer la sécurité des patients (moins de possibilités de prises d'air) et de simplifier la mise en place. Cet appareil pourra être de forme classique rectangulaire ou d'une forme telle qu'il peut être centré autour du pied à sérum et posé sur un support spécial afin d'améliorer la stabilité de l'ensemble.

Dans le présent appareil, tous les instruments de mise en marche, réglage et contrôles (température de sortie du sang, débits des pompes, volumes transfusés, alarmes) sont regroupés sur un même tableau de contrôle. Ainsi, le suivi de la transfusion sera facilité pour le médecin anesthésiste.

L'invention sera bien comprise à la lecture de la description suivante faite en se référant au dessin annexé dans lequel :

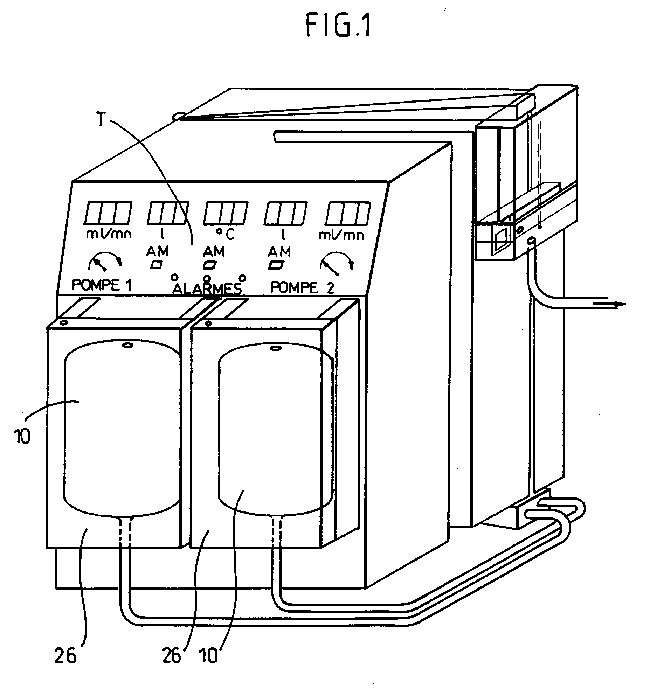

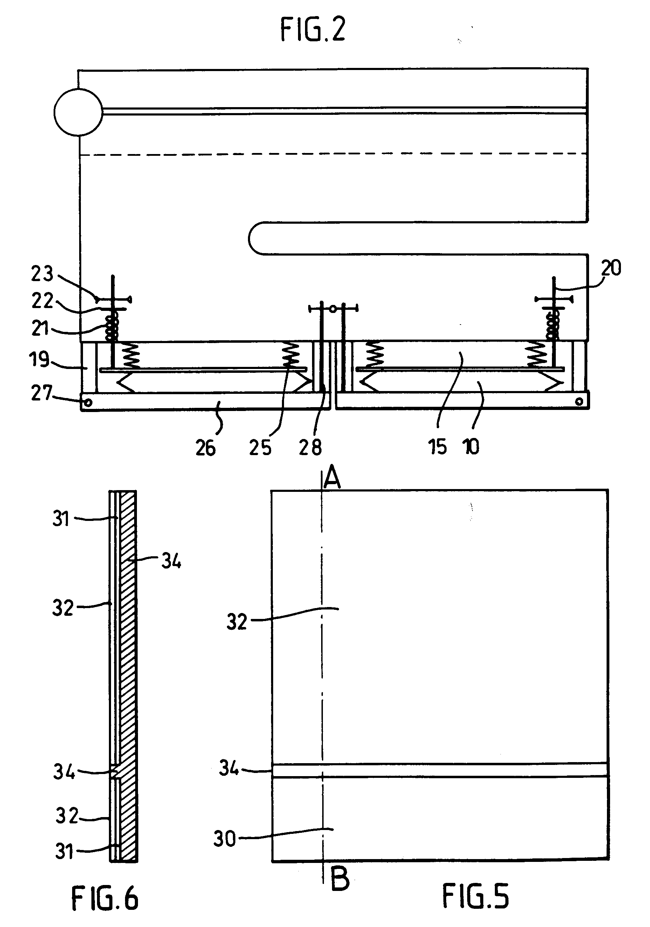

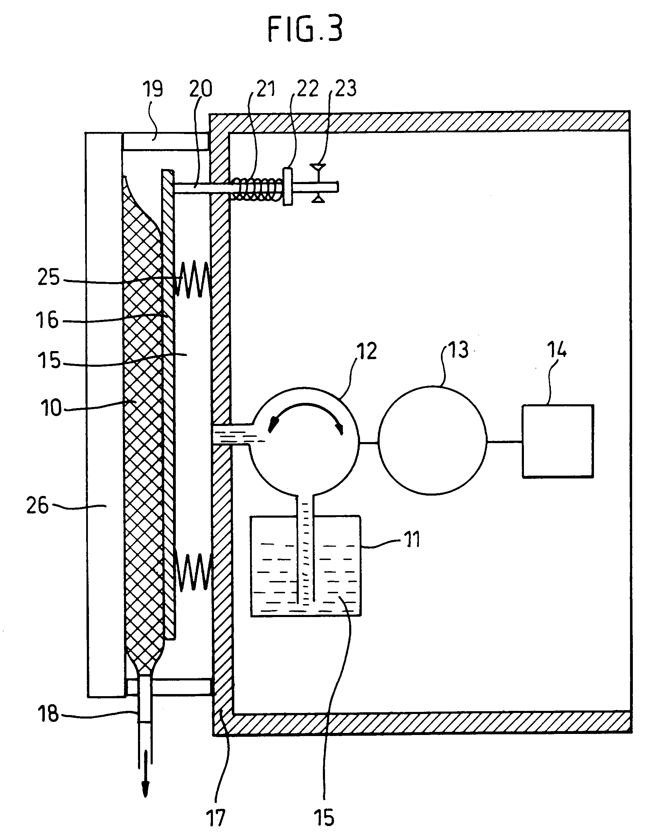

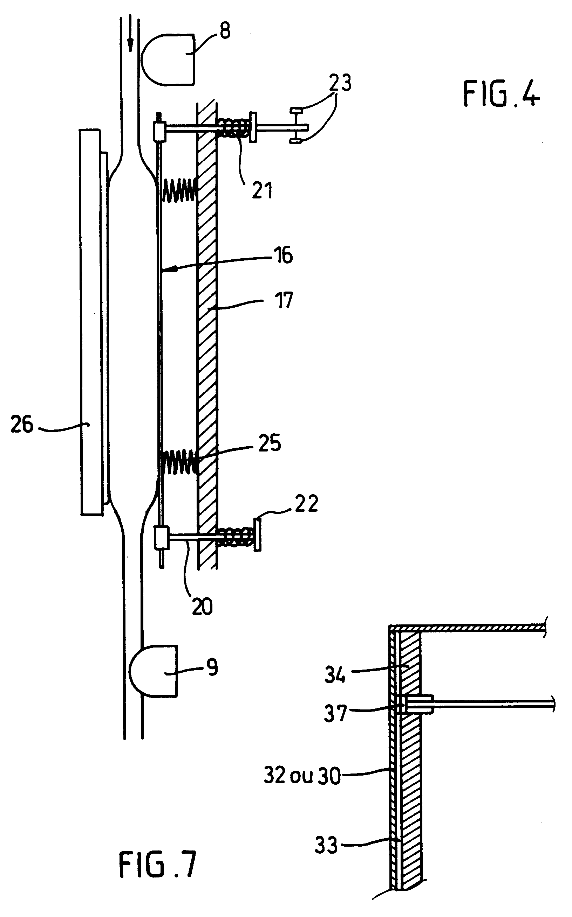

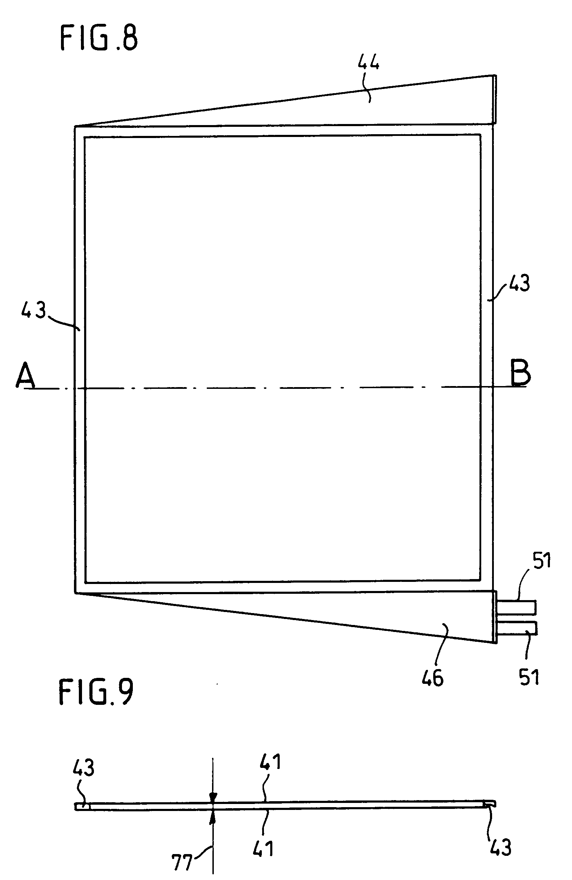

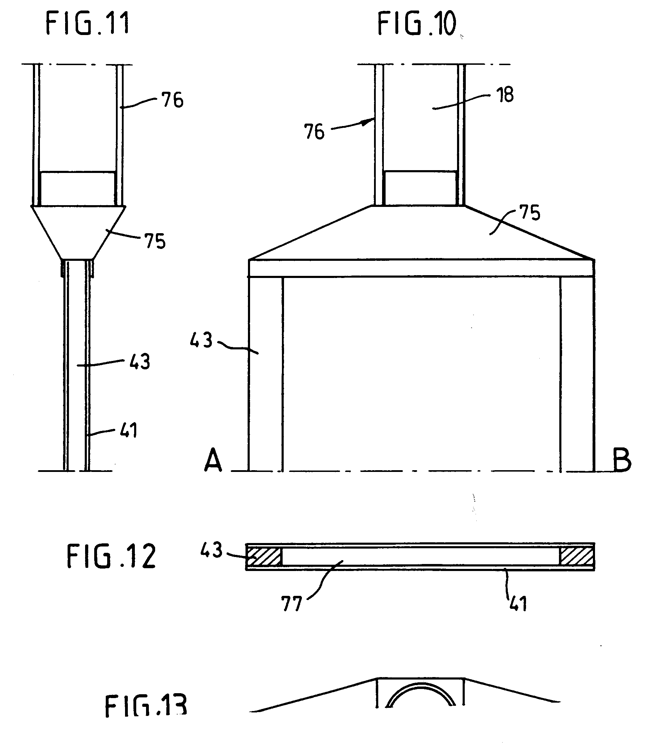

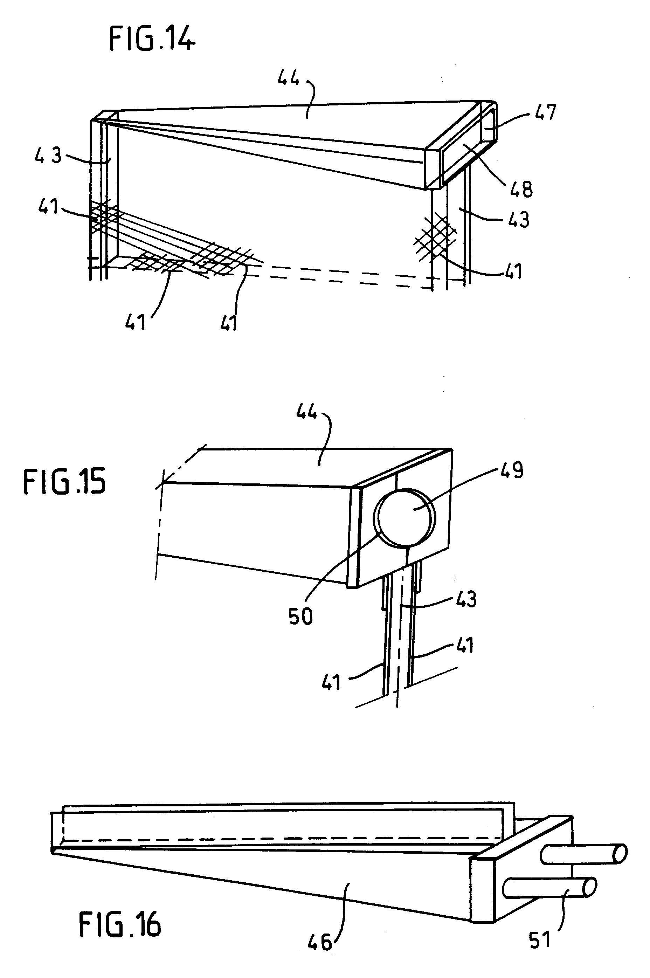

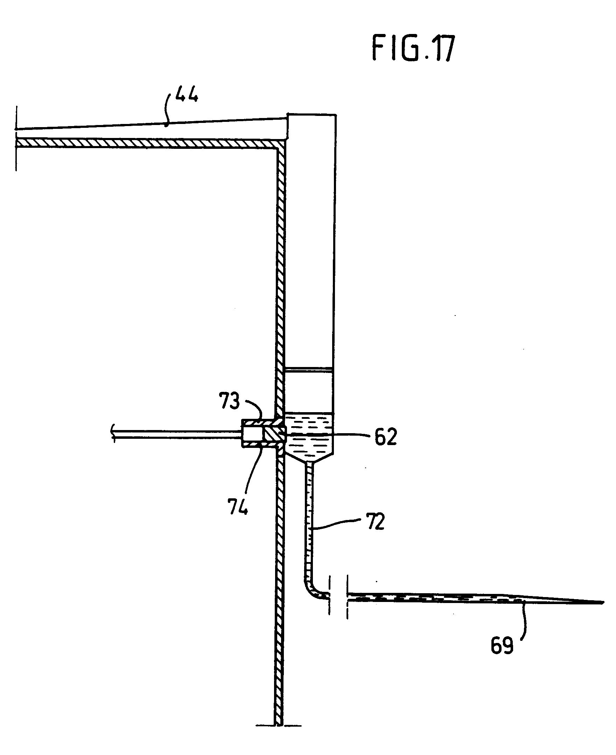

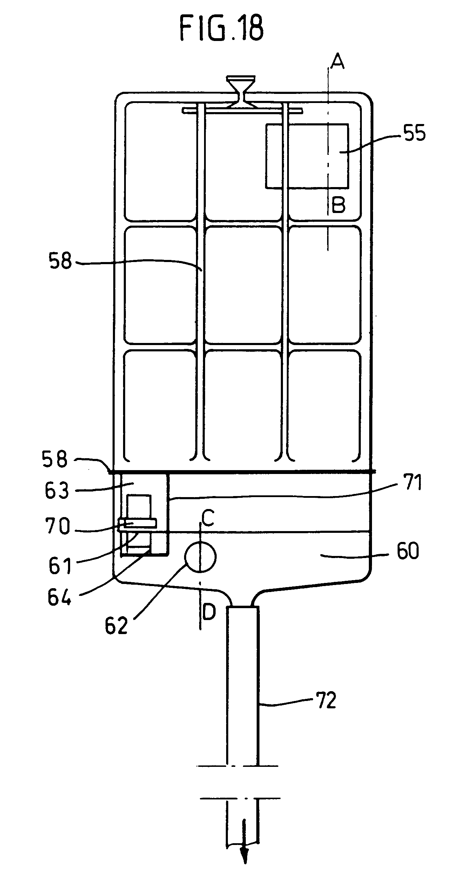

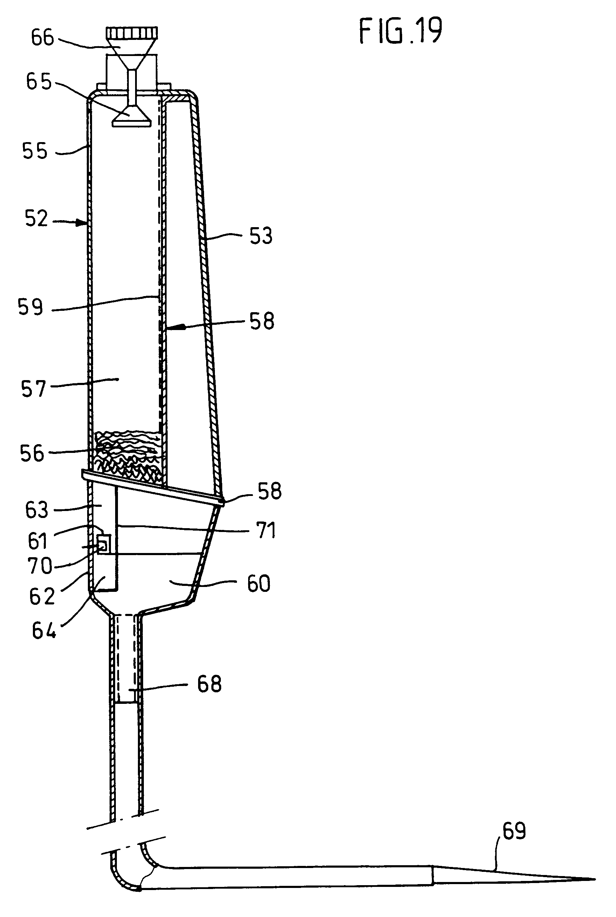

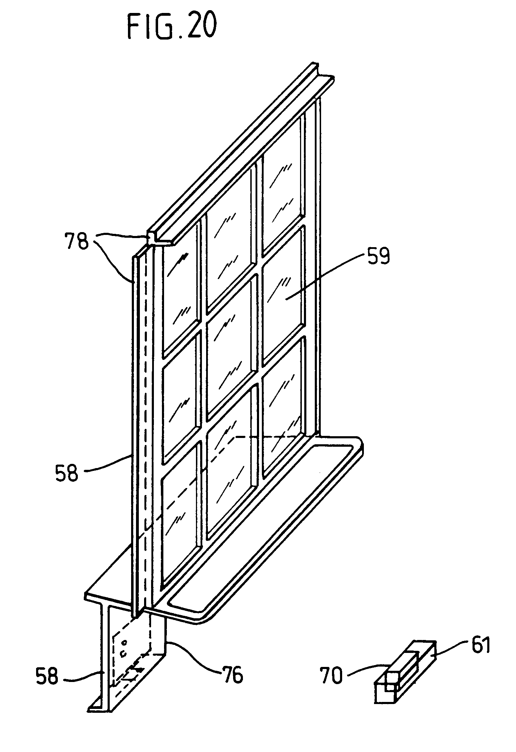

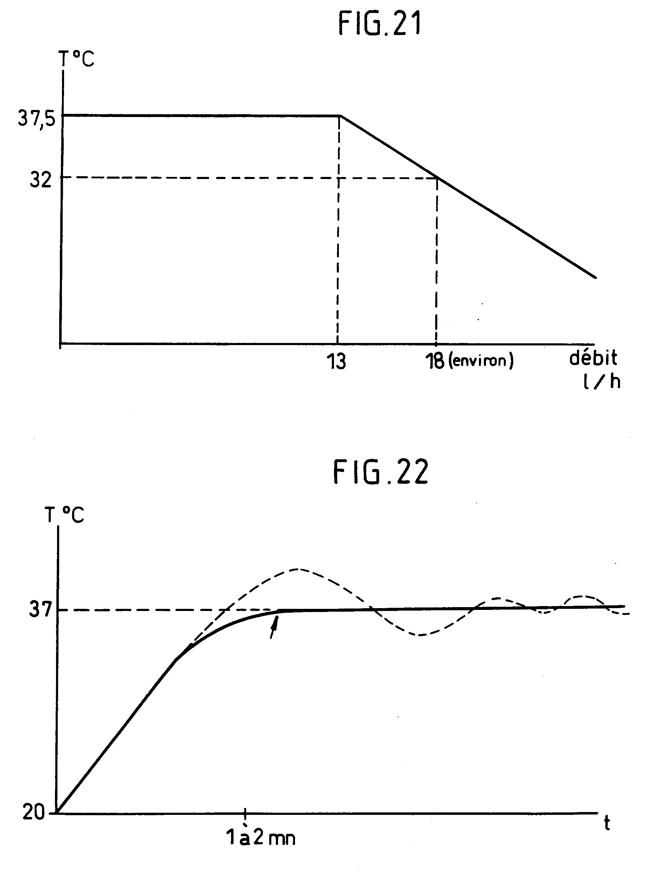

la figure 1 est une vue schématique en perspective d'un appareil de transfusion selon un exemple de réalisation de l'invention; la figure 2 est une vue schématique de dessus de l'appareil de la figure 1; la figure 3 est une vue schématique en coupe représentant une pompe de l'appareil selon l'invention; la figure 4 est une vue d'une partie de la pompe de la figure 3, pour une variante d'utilisation; la figure 5 est une vue en plan des plaques de préchauffage et chauffage disposées de part et d'autre d'un usage unique de la partie réchauffage; la figure 6 est une vue en coupe selon la ligne A-B de la figure 5; la figure 7 est une vue en coupe d'un détail du réchauffeur de sang; la figure 8 est une vue en plan d'un usage unique de la partie réchauffage; la figure 9 est une vue en coupe selon la ligne A-B de la figure 8; la figure 10 est une vue partielle de face d'un usage unique selon une variante; la figure 11 est une vue de profil de l'ensemble de la figure 10; la figure 12 est une vue en coupe selon la ligne A-B de la figure 10; la figure 13 est une vue de dessus de la poche des figures 10 à 12; la figure 14 est une vue en perspective de la partie supérieure de la poche à sang soudée sur son cadre; la figure 15 est une vue en perspective d'une variante de la partie supérieure représentée à la figure 14; la figure 16 est une vue en perspective de la partie inférieure de la poche à sang soudée sur son cadre; la figure 17 est une vue schématique en coupe d'une partie du bas du filtre; la figure 18 est une vue en plan du filtre; la figure 19 est une vue en coupe verticale de l'ensemble de la figure 18; la figure 20 est une vue schématique en perspective du cadre du filtre avec son tamis et son flotteur; la figure 21 représente une courbe des températures de sortie en fonction du débit; et la figure 22 est une courbe de montée en températures en fonction du temps.

On va maintenant décrire les divers composants de l'appareil représenté à la figure 1.

Les pompes utilisées ne sont pas du type péristaltique dont les galets écrasent les hématies et créent une hémolyse du sang par écrasement des globules rouges (hématies) donc dépôt dans le plasma d'une partie de l'hémoglobine. On utilise un appareil nouveau qui peut être appelé pompe hydraulique transfert à compression.

Cet appareil comprend (pour chacune des deux pompes) (figure 3):
- un réservoir 11 empli de liquide incompressible de transfert 15, par exemple de l'eau,
- une pompe rotative ou péristaltique 12 pour transférer ce liquide incompressible dans un réservoir à volume variable 25 du type accordéon,
- un moteur électrique 13 qui commande la pompe 12,
- un variateur électronique de vitesse du moteur 14,
- un réservoir à volume variable 25 relié à la pompe 12 et qui se remplit au fur et à mesure de liquide de transfert,
- un sac 10 de liquide à transfuser séparé du réservoir 25 par une plaque coulissante 16. Cette plaque 16 appuie sur la face interne du sac 10; la face externe de ce sac 10 prend appui sur la face avant 26 de l'appareil. Ladite face 26, de préférence transparente, a une épaisseur importante de l'ordre de 8 à 10mm afin de ne pas se déformer sous la pression exercée par le liquide de transfert 15 dans le réservoir 25 et de rester en une position rigoureusement parallèle à la face avant 17 du boîtier de l'appareil.

Les deux sacs de liquides à transfuser 10 sont fixés sur les deux faces avant ou portes 26 montées pivotantes sur des axes 27. Lorsque les portes 26 sont fermées, leur position est parallèle à la face avant 17 de l'appareil et les portes sont maintenues par des entretoises 19; elles reprennent leur place normale avec précision et les sacs 10 remplis des liquides à transfuser appuient sur le réservoir déformable de compression 25 qui est du type accordéon (soufflet métallique ou poche souple), par l'intermédiaire de la plaque mobile 16 guidée qui exerce la pression sur le sac de sang 10.

Lorsque l'ensemble est en place, le moteur 13 fait tourner la pompe de compression 12 qui remplit progressivement le réservoir déformable 25. Celui-ci, en augmentant de volume, exerce une pression sur les sacs 10 de liquides à transfuser.

L'augmentation de volume du réservoir de compression 25 est proportionnelle au volume du liquide transfusé, donc au débit de la pompe 12. En conséquence, le débit du liquide transfusé est proportionnel à celui de la pompe du liquide de compression et se fait d'une manière continue.

Un microprocesseur à logiciel spécifique pourra indiquer instantanément le débit du liquide transfusé ainsi que son volume, renseignements qui seront affichés numériquement sur le tableau de contrôle T de l'appareil et pourront être transmis à un ordinateur par un interface spécifique.

Les deux portes avant pivotantes 26 comportent une commande automatique d'arrêt du transfuseur lors de l'ouverture, par exemple par l'intermédiaire d'un microrupteur mécanique ou d'un système optoélectronique en sécurité positive.

Dans le cas de soluté utilisé en flacon, on remplira par gravitation un sac classique vide par une entrée de remplissage. Ce remplissage sera aidé par un retour à la position initiale du réservoir à volume variable 25 faisant aspiration lorsque celui-ci reviendra à sa position initiale. Ce retour est aidé par un système à ressorts 21 poussant un axe 20 par sa tête 22, axe fixé sur la plaque intermédiaire 16. Cet axe 20 traverse la face avant 17 du boîtier qui sert d'appui au ressort 21 pour pousser la tête 22 de l'axe 20.

De plus, en fin de compression complète du sac, le fonctionnement de la pompe est inversé de manière à vider le soufflet déformable en transférant le liquide de transfert 15 vers le réservoir fixe 11. Cette commande peut être faite par un système optoélectronique 23 contrôlant le mouvement d'un des axes 20 (ou par un microrupteur) selon que l'axe coupera ou non un rayon lumineux (ou commandera mécaniquement la fermeture ouverture d'un contact électrique).

La pompe peut être utilisée pour la circulation extracorporelle (figure 4). Le sang étant introduit comme indiqué précédemment, un système de cames 8, 9 ferme ou ouvre alternativement les tubulures d'entrée et de sortie. A l'aspiration, la came d'entrée 8 est ouverte et celle de sortie 9 fermée; inversement, lors de la compression, la came 8 d'entrée est fermée et celle de sortie 9 ouverte pour permettre l'évacuation du sang. Les cames 8 et 9 sont commandées chacune par des actionneurs (non représentés).

Le réchauffage du sang ou du concentré globulaire conservé à + 4°C est soumis à deux impératifs :
1/ ne pas dépasser 43°C en montée progressive de température pour éviter l'hémolyse du sang (ou du concentré).
2/ ne pas soumettre brusquement le sang ou le concentré globulaire à une température égale ou supérieure à 38°C. Dans ce cas, il y a risque d'hémolyse par choc thermique.

Dans le dispositif selon la présente invention, on respecte ces deux impératifs par un système de chauffe comprenant deux plaques superposées de chaque côté de la poche, une plaque de préchauffage 30 (figure 5) placée en bas, d'une température inférieure à 38°C (par exemple 36°C) pour éviter l'hémolyse par choc thermique. La température de l'autre plaque 32, isolée thermiquement de la première par une pièce isolante 34, est commandée en fonction de la température de sortie du sang par un capteur de la température de celui-ci, prise à la sortie de l'appareil sur une plaque métallique 62 (figures 17 et 18) avec une sécurité lui interdisant de dépasser 43°C, température d'hémolyse possible du sang. La prise de température pourrait également se faire sur un tube métallique monté sur la tubulure, après le filtre. La poche contenant le liquide à réchauffer est montée entre les deux doubles plaques de préchauffage et de chauffage afin d'augmenter le rendement de celui-ci. Les résistances de chauffe 31 sont montées en sandwich entre les plaques et le support isolant 34.

Avec ce système, la température de sortie du sang sera, dans la tubulure de perfusion, de 37°C et, à surface égale des plaques, on aura une température de sortie de 37°C pour un débit supérieur d'environ 30% puisque, au débit maximum, la température de la plaque de préchauffage et de la plaque de chauffe varieront de 36°/37°C dans le bas (plaque de préchauffage) à 42°C sur la plaque supérieure de chauffe dans les débits les plus importants (au lieu de 18°C à 42°C dans un système traditionnel sans préchauffage).

Dans le présent appareil, la température du sang est donc prise sur la plaque 62 (ou tubulure) métallique en acier inoxydable ou en métal compatible avec le sang, qui sera directement en contact avec le sang réchauffé, par un capteur 73 (figure 17) qui appuie sur la plaque ou le tube fixés à l'extérieur de la poche (sur le côté du piège à air ou sur la tubulure de sortie), ce qui permettra une mesure immédiate du sang réchauffé.

Ce capteur 73 monté sur la paroi latérale et isolé thermiquement par une pièce 74 permet d'indiquer la lecture directement de la température du sang et commande par un ensemble électronique (microprocesseur), le chauffage des plaques supérieures de chauffage 32 selon le principe: proportionnel intégral et différentiel. Cela implique donc que cette plaque de chauffage soit très mince (de manière à avoir une faible inertie thermique) afin que la sortie du sang se fasse à une température de 37,5°C, température idéale pour le patient, qui a toujours tendance à se refroidir au cours de l'opération, d'une part par la perte de sang et d'autre part par la température ambiante de la salle d'opération qui est de l'ordre de 20°C. Un capteur 37 (figure 7) contrôle la température de la plaque de chauffe 32 et arrête celle-ci en cas d'anomalie (température supérieure à 43°C). Le capteur de température 37 pourra être par exemple une thermistance ou tout autre capteur classique (tel que thermocouple ou résistance). Un capteur identique mesure la température de la plaque de préchauffage 30 et la régule à, par exemple, 36 ou 37°C.

Dans les appareils existants, lors des débits importants (supérieurs à 9 litres/heure), la pression peut monter jusqu'à 1 kg/cm². De ce fait les poches en matière plastique de faible épaisseur (0,1 mm à 0,2 mm) ont souvent des fuites soit sur les bordures latérales de celles-ci soit à la jonction de la sortie ou de l'entrée du raccordement de la tubulure avec ladite poche. Cela provient du fait que les sections de sortie et d'entrée de la tubulure sont très inférieures à la section transversale de la poche comprise entre les plaques. Une autre poche connue est composée d'une tubulure plate de faible section (environ 10 mm²) pour des débits importants (9 litres/heure); à cause de cette faible section, la pression est très importante sur les plaques du réchauffeur et sur les côtés latéraux des poches.

Au contraire, conformément à la présente invention, la pression s'appliquant sur les côtés latéraux et sur les plaques de chauffe est très fortement diminuée (de l'ordre de 1/20) par une section de sortie (et d'entrée) égale à la section transversale de la poche. Les avantages sont les suivants :
1/ plus de fuites sur les côtés latéraux,
2/ plus de fuites sur les raccordements des tubulures d'entrée et de sortie des poches, puisque la pression du liquide transfusé est faible,
3/ Le système décrit permet donc d'utiliser ce transfuseur pour des débits beaucoup plus importants, notamment en circulation extracorporelle en utilisant le même principe, pour des débits de l'ordre de 200 litres/heure.

La poche à usage unique selon l'invention est formé simplement de deux feuilles de plastique soudées en haute fréquence. Elle comporte latéralement un cadre rigide ou semi-rigide 43 (figure 8) d'épaisseur égale à l'écart compris entre les plaques de chauffe (ou à cette épaisseur moins deux fois celle des feuilles plastiques de faible épaisseur 41 de 0,1 à 0,2mm), de telle sorte que les feuilles sont comprimées entre le cadre 43 et les plaques de chauffe dont l'écartement est très précis et invariable lorsque l'appareil est fermé, ce qui est facilité par la faible pression interne.

Les cadres latéraux 43 formant entretoises entre les plaques de chauffe sont soudés (ou collés) aux pièces spéciales d'entrée et de sortie. Celles-ci ont une section de sortie égale à la section transversale de la poche. Cela est obtenu par leur forme spéciale (figures 14 à 16). La pièce de sortie (figure 14) est soudée aux cadres et aux deux feuilles latérales; sa face supérieure 44 est telle qu'elle va en s'évasant vers le côté sortie 48 et se termine par une pièce de section carrée 47 (figure 14) ou une pièce 50 à sortie circulaire 49 (figure 15) et se raccordant avec le filtre décrit ci-après qui fait partie de l'usage unique complet.

La pièce inférieure d'entrée 46 (figure 16) a une section de même dimension que la pièce de sortie. Elle est soudée aux deux feuilles latérales et aux cadres. Elle a une forme semblable à celle de la pièce supérieure. L'entrée comprend une pièce (de formage ou de moulage) ayant deux tubes (figure 16) sur lesquels deux tubulures 51 se terminant par un cône Luer sont raccordées aux sacs des liquides à transfuser.

Les entrées et sorties du sang peuvent également se faire axialement (voir figures 10 à 13), notamment pour des débits importants tels que ceux utilisés en circulation extra-corporelle. Les pièces d'entrée et de sortie 75 s'évasent des côtés vers le centre jusqu'à la tubulure 76 qui a une section égale à la section transversale 77 de la poche comprise entre les plaques de chauffe.

Le filtre est du type décrit dans US-A-4.320.001 mais il comporte les améliorations suivantes:
- la partie inférieure est continuée après le tamis de micro-filtration de sorte que le sang (arrivant par une partie évidée 55 (figures 18-29) après la partie filtration 59 proprement dite tombe dans un second volume 60 formant piège à air. Dans ce piège, un flotteur 61 comportant un aimant 70 permet par un contrôle magnétique (ou par une détection optoélectronique) d'arrêter le fonctionnement du transfuseur dans le cas des deux anomalies suivantes.

1/ clampage aval de la tubulure: dans ce cas, le sang monte dans le piège et le flotteur et s'arrête en partie haute 63.
2/ si pour une raison quelconque, l'air est introduit à la place du sang, le flotteur se placera à la partie inférieure 64 du piège à air. Il est guidé par les parois 71 (figure 18) et 76 (figure 20).

Sur le côté du piège à air est fixée une plaque métallique de faible épaisseur 62 (figures 18 et 19) par exemple de 0,5mm, en acier inoxydable dans la paroi ajourée 20 (ou tout autre produit conducteur de la chaleur et compatible avec le sang). En fonctionnement normal de l'appareil, cette plaque sera chauffée par le sang qui est dans le bas du piège à air d'un côté et appuiera de l'autre sur un capteur de température 73 (figure 17) (thermistance, résistance ou thermocouple) qui commande et régule la température des plaques supérieures 32 entre 37°C et 42°C de telle manière que le sang à la sortie du filtre soit à la température de 37°C dans la zone d'utilisation prévue. Le système de régulation est classique (microprocesseur) mais du type à régulation: proportionnelle, intégrale et différentielle, ce qui permet une courbe régulière sans dépassement de température (figure 22).

Le raccordement de ce filtre sur la partie supérieure de sortie du sang se fait par la pièce de sortie de la poche 47 qui s'encastre dans l'ajourage du filtre 55. La sortie du filtre est raccordée par une tubulure classique 72 d'environ 1,20 mètre et se terminant par le trocart de perfusion 69 (figure 19).

## Revendications

1. Appareil de transfusion sanguine du type comprenant :
- une alimentation en sang, par exemple sous forme d'au moins un sac de concentré globulaire ou un sac de plasma ou de soluté ou d'albumine,
- au moins une pompe accélératrice de sang,
- une poche à usage unique pour la pompe,
- un réchauffeur de sang,
- une poche à usage unique pour le réchauffeur,
- un système de filtration,
- une ligne de perfusion raccordée par des embouts coniques et terminée par un trocart,
caractérisé en ce que la pompe accélératrice comprend un volume variable (25) du type accordéon ou poche souple et un sac (10) de liquide à transfuser disposés entre deux plaques (17, 26) à écartement précis, avec entre le volume variable (25) et le sac (10) un plateau (16) coulissant parallèlement aux plaques (17, 26), le volume variable (25) étant alimenté par une pompe (12).

2. Appareil selon la revendication 1,
caractérisé en ce que la poche à usage unique pour le réchauffeur comprend une pièce d'entrée (46) et une pièce de sortie (44) telles que la section de passage (48) du liquide soit égale à la section transversale (77) de la poche, un cadre (43) formant entretoise ayant la même épaisseur que l'intervalle entre les plaques de chauffe (32) et auquel sont soudées des feuilles (41) de faible épaisseur délimitant la poche.

3. Appareil selon l'une des revendications 1 et 2,
caractérisé en ce que le système de filtration comprend un tamis de micro-filtration et est prolongé par un piège à air comprenant un flotteur (61) et un moyen de détection, tel qu'un aimant (70), pour détecter la position dudit flotteur (61) et commander l'arrêt de l'appareil en cas d'anomalie.

4. Appareil selon la revendication 3,
caractérisé en ce qu'une pièce (62) transmettant la chaleur est fixée dans la paroi plane du filtre en appui sur un capteur de température (73) pour permettre de mesurer la température de sortie du liquide et de réguler le réchauffeur.

5. Appareil selon l'une des revendications 1 à 4,
caractérisé en ce que le réchauffeur comprend un ensemble de deux plaques doubles comportant chacune une plaque de préchauffage (30) à température constante légèrement inférieure à 38°C et une plaque de chauffe (32) à température variable commandée par un capteur de température (73) de sortie du liquide.

## Claims

1. Blood transfusion apparatus of the type comprising:
- a blood supply, for example in the form of at least one bag of concentrated corpuscles or a bag of plasma or of solution or of albumin,
- at least one blood accelerating pump,
- a single-use bag for the pump,
- a blood reheater,
- a single-use bag for the reheater,
- a filtration system,
- a perfusion line connected by conical couplings and terminating in a trocar,
characterized in that the accelerating pump comprises a variable volume (25) of the accordion or flexible pocket type and a bag (10) of liquid to be transfused disposed between two plates (17, 26) precisely spaced apart, with between the variable volume (25) and the bag (10) a platen (16) sliding parallel to the plates (17, 26), the variable volume (25) being supplied by a pump (12).

2. Apparatus according to claim 1,
characterized in that the single-use bag for the reheater comprises an inlet member (46) and an outlet member (44) such that the section of passage (48) for the liquid will be equal to the transverse cross section (77) of the bag, a frame (43) forming a stay having the same thickness as the interval between the heating plates (32) and to which are sealed sheets (41) of small thickness delimiting the bag.

3. Apparatus according to one of claims 1 and 2,
characterized in that the filtration system comprises a micro-filtration screen and is prolonged by an air trap comprising a float (61) and a detection means, such as a magnet (70), to detect the position of said float (61) and to control the stopping of the apparatus in the case of malfunction.

4. Apparatus according to claim 3,
characterized in that a member (62) transmitting heat is fixed in the flat wall of the filter bearing on a temperature detector (73) to permit measuring the outlet temperature of the liquid and to adjust the reheater.

5. Apparatus according to one of claims 1 to 4,
characterized in that the reheater comprises an assembly of two double plates each comprising a preheating plate (30) of constant temperature slightly less than 38°C and a heating plate (32) at a variable temperature controlled by a temperature detector (73) at the outlet of the liquid.

## Patentansprüche

1. Bluttransfusionsvorrichtung der Bauart mit:
- einer Zufuhrvorrichtung von Blut, z.B. in Form wenigstens eines Beutels von Zellkonzentrat, eines Beutels mit Plasma, mit einem gelösten Stoff oder mit Albumin,
- wenigstens einer Pumpe zur Beschleunigung des Blutes,
- einer Einwegtasche für die Pumpe,
- einer Vorrichtung zum Aufwärmen des Blutes,
- einer Einwegtasche für die Vorrichtung zum Aufwärmen des Blutes,
- einem Filtrationssystem,
- einer Infusionsleitung, die durch konische Ansatzstücke angeschlossen ist und mit einem Trokar endet, **dadurch gekennzeichnet**, daß die Pumpe zur Beschleunigung ein variables Volumen (25) der Bauart eines Akkordeons oder einer flexiblen Tasche und einen Beutel (10) mit Transfusionsflüssigkeit umfaßt, die zwischen zwei Platten (17, 26) im genauen Abstand angeordnet sind, mit einer Scheibe (16) zwischen dem variablen Volumen (25) und dem Beutel (10), die sich parallel zu den Platten (17, 26) verschiebt, wobei das variable Volumen (25) von einer Pumpe (12) gespeist wird.

2. Vorrichtung nach Anspruch 1,
**dadurch gekennzeichnet**, daß die Einwegtasche für die Vorrichtung zum Aufwärmen ein Eingangselement (46) und ein Ausgangselement (44) umfaßt, die so sind, daß der Querschnitt des Durchtritts (48) der Flüssigkeit dem Querschnitt (77) der Tasche entspricht, wobei ein Rahmen (43) ein Zwischenstück bildet, das die gleiche Dicke aufweist wie der Abstand zwischen den Heizplatten (32), und an dem Folien (41) geringer Dicke geschweißt sind, die die Tasche abgrenzen.

3. Vorrichtung nach einem der Ansprüche 1 und 2,
**dadurch gekennzeichnet**, daß das Filtrationssystem ein Sieb zur Mikrofiltration umfaßt und durch eine Luftfalle verlängert ist, die einen Schwimmer (61) und ein Mittel zum Aufspüren, wie einen Magneten (70), umfaßt, zur Ermittlung der Position des Schwimmers (61) und zur Steuerung des Stillstandes der Vorrichtung beim Auftreten von Anomalien.

4. Vorrichtung nach Anspruch 3,
**dadurch gekennzeichnet**, daß ein Element (62), das die Wärme überträgt, in der ebenen Wand des Filters in Anlage auf einem Temperatursensor (73) befestigt ist, um die Austrittstemperatur der Flüssigkeit messen zu können und um die Vorrichtung zum Aufwärmen zu steuern.

5. Vorrichtung nach einem der Ansprüche 1 bis 4,
**dadurch gekennzeichnet**, daß die Vorrichtung zum Aufwärmen eine Baugruppe von zwei doppelten Platten umfaßt, von denen jede eine Platte zum Vorwärmen (30) auf konstante Temperatur etwas unterhalb von 38 °C und eine Platte zum Aufwärmen (32) auf variable Temperatur umfaßt, die von einem Temperatursensor (73) am Austritt der Flüssigkeit geregelt wird.
